# EUROPEAN PATENT APPLICATION

(11) **EP 1 010 763 A1**
(43) Date of publication of application: **21.06.2000**
(21) Application number: 98403135.1
(22) Date of filing: 11.12.1998
(51) Int. Cl.: C12N 15/67, C12N 15/12, C12N 9/12, C12N 15/52

(54) **Enhanced expression of heterologous proteins in recombinant bacteria through reduced growth temperature and co-expression of rare tRNA's**

(71) Applicant: INSTITUT PASTEUR, 75724 Paris Cédex 15 (FR)
(72) Inventor: Papanicolaou, Catherine, 75009 Paris (FR); Rougeon, François, 78125 Poigny-La-Forêt (FR); Boule, Jean Baptiste, 75011 Paris (FR)
(74) Representative: Le Guen, Gérard

(57) **Abstract**

This invention concerns a method for large scale production and isolation of heterologous proteins expressed in bacteria, by coupling low growth temperature to overexpression of rare tRNAs.

## Description

The present invention relates to a method for large scale production of soluble integral heterologous proteins in bacteria.

Errors accompanying translation, frame shifts and misincorporations, have been widely described in all types of biological systems (Parker, 1989). In the case of heterologous protein expression, these errors can have repercussions on both the level of expression and on the integrity of the recombinant protein (Kurland and Gallant, 1996). Kurland and Galland exhort the biotechnology community to devote more emphasis to the determination of level of misincorporation. All too often, when a protein is well expressed, soluble, active and corresponds to the expected molecular weight, the integrity of the protein is no longer questioned. In 1996 Olins notes that the drive for high expression levels unfortunately commonly leads to lower quality or authenticity of the protein's sequence or structure. Olins further notes that even using the most sophisticated analytical techniques currently available, altered recombinant proteins can only be detected when they are present at a level of ≥1%. Recent work has shown that misincorporation rates, at certain positions, may be as high as 40%. These positions correspond to codons which are said to be rare; i.e., frequent in the donor species but rare in the host organism (Calderone et al., 1996) . Table 1 hereafter, taken from Zhang et al., (1991) summarizes, for each of 3 species (bacterium *Escherichia coli*, yeast *Saccharomyces cerevisiae*, fruit fly *Drosophila melanogaster*) and for primates (8 different species), the eight least utilized codons.

This species-dependent codon usage is of theoretical interest and has been intensively studied (Zhang et al., 1991 ; Bagnoli and Lio, 1995). However, it is still often overlooked when it comes to large scale heterologous protein production (Kane, 1995 ; Kurland and Gallant, 1996). When post-translational modifications not made in prokaryotes are unessential to the activity of the recombinant protein, the usual choice of host for the expression of eukaryotic genes is *Escherichia coli*. This is true for practical reasons such as cost and simplicity, as noted by Olins (1996). *E. coli* is however quickly bypassed for other systems of expression once problems, such as low production or inclusion body formation, are encountered. These problems may be due to translation errors leading to a saturation of chaperone proteins while such translation errors may in turn be due to a high prevalence of low usage codons. Alternative expression systems, such as those in yeast, baculovirus, and vaccinia virus, may themselves pose other problems and do not always offer a solution to problems of output and solubility, particularly when they are related to codon usage (see Table 1).

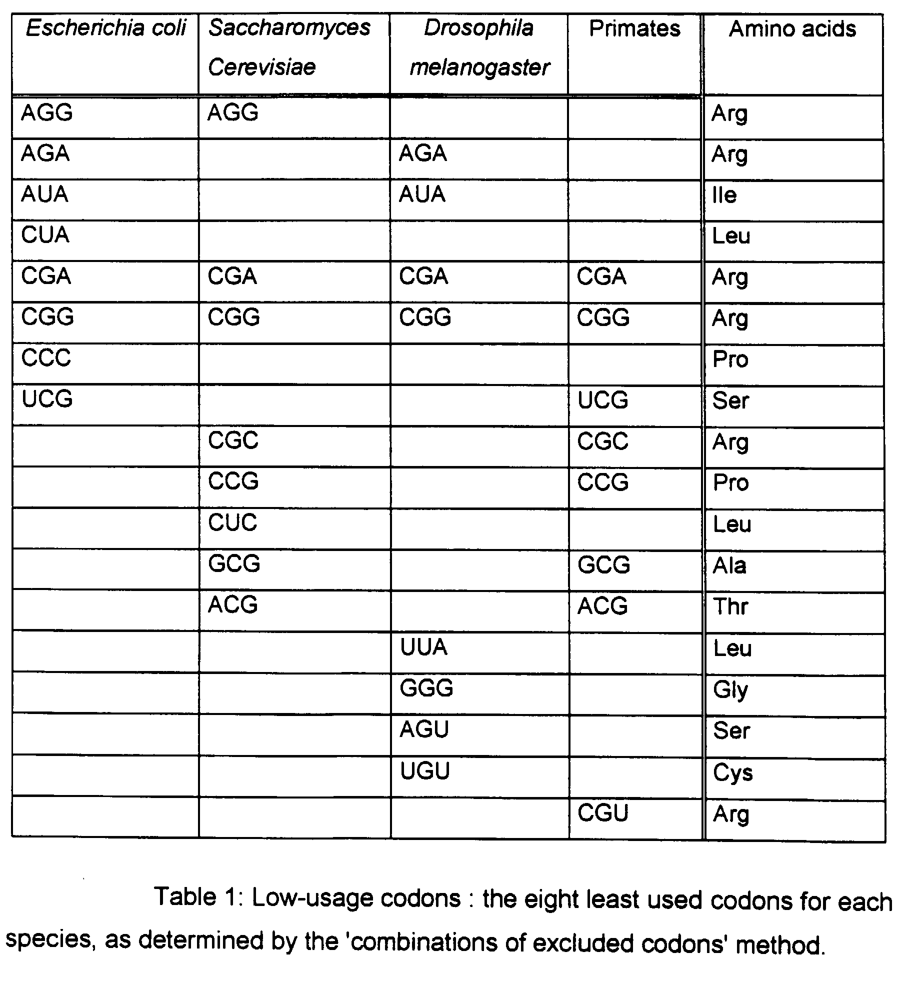

A review of codon frequencies in *E. coli* shows that the two arginine codons AGA and AGG are the rarest of all (0.14 and 0.21 %, respectively) (Wada et al., 1992). At positions which correspond to isolated AGA codons, an abundant misincorporation of lysine was shown in one heterologous protein (Calderone et al., 1996). It was also shown that the presence of these two codons, in tandem or concentrated in a motif, reduced the translation of certain proteins (Kane, 1995), in a drastic manner (Kane, 1995) and that, when present in tandem, AGG and AGA led to 50 % translation frameshifts (Spanjaard, 1988). These frameshifts could be suppressed with the overexpression of the *argU* gene coding for a rare tRNA in *E. coli* (Spanjaard, 1990). Finally, the rate of synthesis for two proteins rich in isoleucine coded by AUA (the third rarest codon in E. coli) could be improved by overexpression of the *ileX* gene (Del Tito, 1995).

Because of the existing prior art, the authors of the present invention have been interested in obtaining the expression in a bacterial host of soluble and integral forms of eukaryotic proteins.

For example, no soluble terminal deoxynucleotidyl transferase (TdT), even in a truncated form, has ever been produced in bacteria and neither full length RAG1 nor full length RAG2 protein (encoded by Recombination Activating 1 and 2 Genes) has been obtained with standard heterologous expression systems.

Indeed, in 1988, Chang et al overproduced human terminal transferase (TdT) by cloning the coding sequence of human TdT into a baculovirus. The authors of this article had selected the baculovirus system because their attempts to overproduce this protein in *E. coli* were not successful. Yang et al in 1994 used human TdT that was overexpressed in a baculovirus system for mutational analysis of residues in the nucleotide binding domain of this protein. However this baculovirus system has been disappointing and has never allowed the complete description of the enzyme. Consequently, there is a need for an alternative method for large scale production and isolation of TdT.

Due to a lack of solubility of the full length proteins, Mc Blane et al, in 1995, were only able to produce truncated versions of RAG1 and RAG2 proteins. The use of these truncated proteins has permitted the reconstitution of basic V(D)J recombination in an acellular system (Ramsden et al, 1997). However, recent works (Roman et al, 1997 ; Kirch et al, 1998), where evidences for an *in vivo* essential role of the deleted portions of both RAG1 and RAG2 are presented, emphasize the need to obtain the full length proteins in order to make use of their potential in acellular systems.

The authors of the present invention have proposed that the reason that such heterologous proteins could not be produced in their integrity was their content in rare codons, i.e. codons that are present at a low frequency in the genome of the host. For example, the deleted portion of the cDNA coding for murine TdT contains three tandems of rare arginine codons in *E. coli*. The low frequency of such rare codons in the genome of the host is accompanied by a poor expression of corresponding tRNAs.

The authors of the present invention have shown that overexpression of a rare tRNA improves TdT, RAG1 and RAG2 proteins output but that the proteins remain in insoluble aggregates.

In 1989, Schein reported that the production of recombinant proteins in bacteria is limited by the formation of cytoplasmic aggregates and that one way to lower the formation of inclusion bodies is to reduce the growth temperature of the bacteria. Temperatures down to 25°C are used.

La Vallie et al have described, in 1993, a bacterial expression system, where the heterologous proteins are fused to thioredoxin in order to improve their solubility. The accumulation in solution of certain proteins is reported to be facilitated by low growth temperatures (25 or 15°C).

By using very low growth temperatures (inferior or equal to 20°C) to overexpress a rare tRNA along with the heterologous protein, the authors of the invention have been able to circumvent all prior technical handicaps and produce large quantities of heterologous proteins in their soluble form, thus allowing easy isolation and purification.

The present invention more particularly relates to a method for producing a soluble integral heterologous protein in a bacterium,
wherein said protein contains amino acids encoded by codons that are rare in the genome of said bacterium and said bacterium does not naturally contain a sufficient amount of tRNA corresponding to said rare codons to allow the production of said complete heterologous protein ;
said method comprising the steps of :
a) transforming said bacterium with i) a nucleic acid encoding at least a tRNA that is rare in said bacterium and that corresponds to at least said rare codon, so as to overexpress said tRNA ; and ii) a nucleic acid encoding said heterologous protein ;
b) culturing said bacterium at a temperature inferior to the usual growth temperature, whereby large quantities of said heterologous protein are produced in an integral and soluble form ;
c) extracting said heterologous protein from said bacterium ; and
d) optionally purifying said heterologous protein.

The term "nucleic acid *encoding* a tRNA" means a nucleic acid from which a tRNA precursor transcript is produced. Said nucleic acids are well-known by one skilled in the art. For instance, the *Escherichia coli argU* gene encodes a monocistronic tRNA precursor transcript which is processed at both 5' and 3' ends to form arginine tRNA^{ARG}_{UCU} (Garcia et al, 1986).

The term "heterologous protein" designates a protein that is naturally expressed in an organism that is different from the host organism, i.e any standard bacterial expression system, e.g. *Escherichia coli*.

Such heterologous proteins may be for example terminal deoxynucleotidyl transferase (TdT), RAG1 and RAG2 proteins (encoded by Recombination Activating 1 and 2 Genes).

The term "integral protein" designates a protein for which translation is sufficiently accurate to allow said protein to exhibit the biological activity known for the natural protein.

The variants of these proteins are also encompassed. The term "variant protein" means an engineered derivative form of the natural protein. The variants can exhibit substantially the same or an improved biological activity.

The term "usual growth temperature" means the standard temperature at which cell growth is optimal as well-known by one skilled in the art. For example, usual growth temperature for a bacteria such as *E. coli* is about 37°C.

The authors of the present invention have surprisingly discovered that the step of culturing said bacteria can be advantageously carried out at a temperature much lower than the usual growth temperature. The selected temperature is preferably not less than 4°C, preferably not less than 10°C and not more than 20°C, preferably not more than 15°C. The selected temperature is preferably between 4°C and 20°C.

This low temperature improves the yield of soluble integral heterologous proteins produced by the above method.

According to the method of the invention, the bacterium is transformed with i) a nucleic acid encoding at least a tRNA that is rare in said bacterium and that corresponds to at least said rare codon so as to overexpress said tRNA; and ii) a nucleic acid encoding said heterologous protein.

Said nucleic acids are placed under the control of the elements required for transcription, in particular under the control of an appropriate promoter.

The nucleic acid encoding the heterologous protein is more particularly inserted in an expression cassette. The choice of the expression cassette depends on the selected host system. Typically, an expression cassette includes a promoter that is functional in the selected host system and can be constitutive or inducible ; a ribosome binding site ; a start codon (ATG) if necessary, a region encoding a signal peptide, e.g, a periplasmic localization signal peptide ; a DNA molecule coding for an heterologous protein ; a stop codon ; and optionally a 3' terminal region (translation and/or transcription terminator).

The expression cassette is typically part of an expression vector which is selected for its ability to replicate in the chosen expression system.

Expression vectors (e.g., plasmids or viral vectors) can be selected from those described in Pouwels et al (cloning vectors : a Laboratory Manual, 1985, s upp. 1987).

Said nucleic acids (encoding a tRNA and encoding a heterologous protein) may be inserted in one vector or in two different vectors.

The various methods for transforming a bacterial host with said nucleic acids are well-known by any one skilled in the art. Typically, one can use electroporation.

In order to produce large quantities of two different forms of soluble TdT, the authors of the present invention have transformed two *E. coli* strains, and said transformed strains were deposited at the CNCM on February 10, 1998 under the file number I-1981 and I-1982.

Strain I-1981 produces murine TdT deleted of amino acids 2-129.

Strain I-1982 produces full murine TdT.

In order to produce murine RAG1 and RAG2 proteins, the authors of the present invention have transformed two *E. coli* strains, and said transformed strains were deposited at the CNCM on October 22, 1998, under the file numbers I-2086 (producing RAG1) and I-2087 (producing RAG2).

The extraction and purification methods of the soluble recombinant proteins are well-known by one skilled in the art. However these methods were never before successfully employed for TdT and full-length RAG1 and RAG2, produced in a bacterium, as disclosed in the present invention. For instance, a recombinant protein can be recovered from the host cell extract or from the supernatant after centrifugation of the recombinant cell culture.

Typically, the soluble heterologous protein can be purified by antibody-based affinity purification or by any other method that can be readily adapted by one skilled in the art, such as by genetic fusion to a small affinity binding domain.

The present invention further concerns a recombinant soluble and integral TdT protein produced in bacteria, as well as a recombinant soluble and integral RAG1 and RAG2 proteins produced in bacteria.

The following figures and examples illustrate the present invention without reducing the scope of protection in any way.

### Legends of the figures

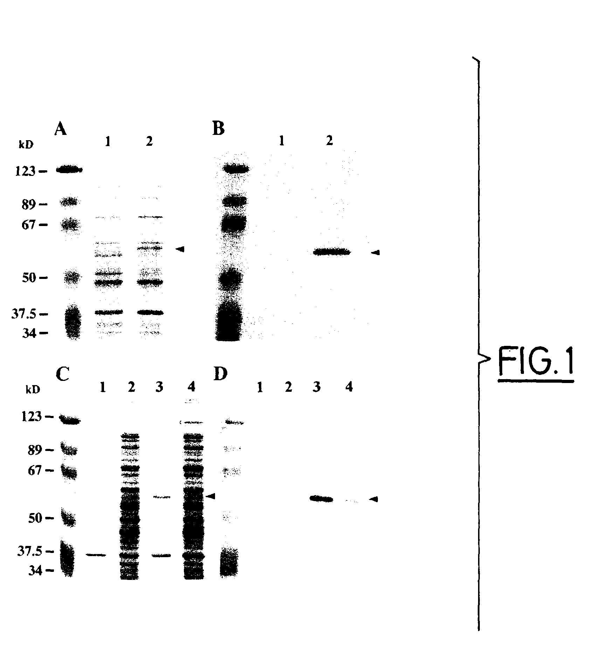

Figure 1. **Expression of murine TdT in *E.coli.*** Coomassie staining (panel A) and immunoblot (panel B) analyses of crude extracts prepared from bacterial cultures after 3 hours IPTG induction. Total bacterial extracts were separated on a 8% gel by SDS-PAGE. After electrophoretic separation, half of the gel was stained directly with Coomassie Blue. Proteins present in the other half of the gel were transferred onto a nitrocellulose sheet and tested for reaction with a rabbit serum raised against bovine TdT. Immunocomplex was detected by reaction with goat anti-rabbit alkaline phosphatase conjugate followed by development in 100 mM Tris pH 9.5, 100 mM NaCl, 165 µg/ml BCIP, 330 µg/ml NBT. Figure 1, Panel A, lane 1, shows proteins present in *E.coli* BL21 DE3 cells transformed with the pET22b expression vector; lane 2, proteins present in the same cells transformed with the murine TdT c-DNA containing pET22b vector (pET22-TdTS). Panel B, lanes 1 and 2, corresponding immunoreactive material; Coomassie staining (panel C) and immunobloting (panel D) of pellet and supernatant fractions obtained from bacteria transformed with pET22b (lanes 1-2) and pET22b-TdTS (lanes 3-4). Cells were lysed and centrifuged at high speed to pellet the insoluble proteins. Panel C, lane 1 and 3, insoluble proteins and lane 2 and 4, soluble proteins. Panel D, lanes 1-4, corresponding immunoreactive material. Arrows indicate the position of migration of recombinant murine TdT. Molecular weight markers are displayed on the left side of the gels.

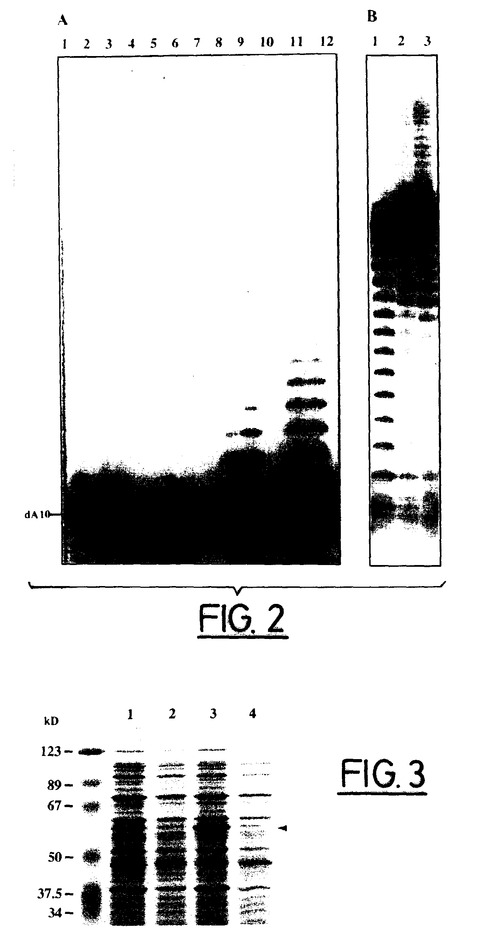

Figure 2. **Analysis of TdT activity in bacterial extracts.**
   This figure shows the assay of soluble and insoluble fractions for terminal transferase activity by incorporation of dGTP (1mM initial concentration) on a [³²P]-labelled (dA)₁₀ (1µM), in 200 mM potassium cacodylate, 25 mM Tris pH 6.6, 250 mg/ml BSA, in the presence of 1.2 mM Co⁺⁺. Products were analysed on a 16 % acrylamide denaturing gel and chain length distribution was visualized by autoradiography. A time course reaction (0, 5 and 15 min) is shown for each experiment. Panel A, assay with proteins produced at 37°C (2-6 µg of extract). Activities are displayed for pellet (lanes 1-3) and supernatant (lanes 4-6) of bacterial extract containing pET22b, and pellet (lanes 7-9) and supernatant (lanes 10-12) of bacterial extract containing pET22b-TdTS. Panel B, assay with proteins produced at 15°C (10 µg of extract). Lanes 1-3, activities in supernatant of bacterial extract containing pET22b-TdTS.
Figure 3. **Effects of overexpression of a rare arginine t-RNA on the production level of recombinant TdT.** Crude extracts (lanes 1 and 3) and supernatant fractions (lanes 2 and 4) prepared from bacteria expressing pET22-TdTS (see Experimental Procedures) were analysed by SDS PAGE and Coomassie staining. Lanes 1 and 2, proteins recovered from cells that do not express *argU* t-RNA; lanes 3 and 4, proteins recovered from cells coexpressing *argU* t-RNA.

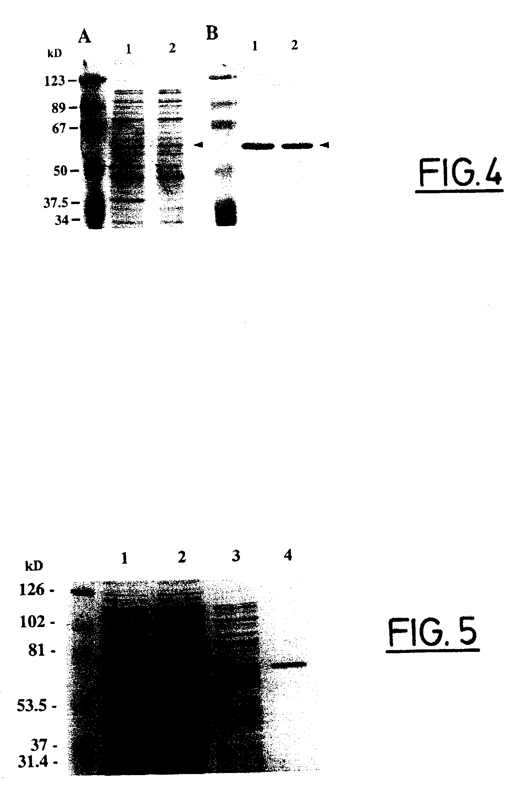

Figure 4. **Solubility of murine TdT produced at 15°C.** Crude extract and supernatant fraction prepared from bacteria coexpressing pET22-TdTS and *argU* tRNA at 15°C were analysed by SDS-PAGE. Coomassie staining and Immunoblotting are shown in panel A and B respectively. Panel A; lane 1, crude extract; lane 2, supernatant. Panel B; corresponding immunoreactive material.
Figure 5. **Purification of His-tagged murine TdT by affinity chromatography.** Aliquots taken at each step of the purification on a Ni⁺⁺ chelating column were analysed by SDS-PAGE and Coomassie staining. Lane 1, crude extract; lane 2, column flow through; lane 3, 60 mM Imidazole wash; lane 4, 500 mM Imidazole elution.

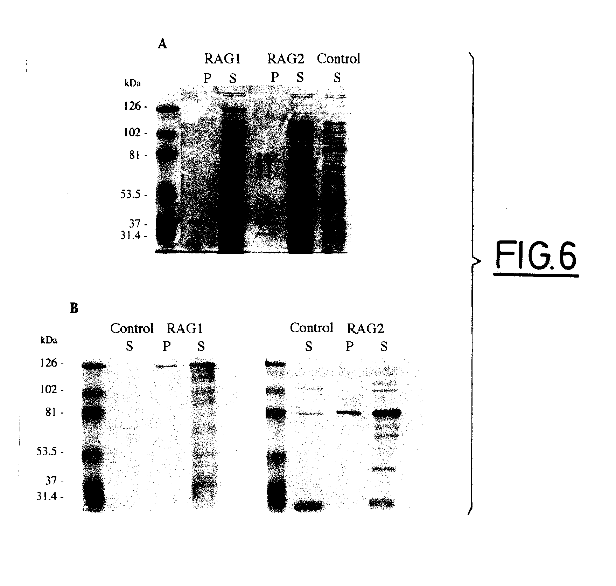

Figure 6. **Solubility of recombinant murine full length RAG1 and RAG2 proteins**. Pellet (P) and supernatant (S) fractions for RAG1 and RAG2 expressing cells are shown adjacent to the supernatant fraction of a control bacterial lysate. (Coomassie staining, panel A ; immunoblotting, panel B).

### Example 1: Production of soluble, integral murine TdT in E. coli

### 1. Introduction

Terminal deoxynucleotidyl transferase activity was discovered in 1960, in calf thymus gland (Bollum et al, 1960). The enzyme was purified to apparent homogeneity in 1971 (Chang et al, 1971), and its catalytic properties extensively investigated in the following years (Bollum et al, 1974 ; Coleman et al, 1977). Terminal deoxynucleotidyl transferase (EC 2.7.7.31; TdT) is a template-independent polymerase which catalyzes the random addition of deoxynucleoside 5'-triphosphates onto the 3'-hydroxyl group of a single stranded DNA initiator in a stepwise (distributive) rather than processive fashion (Chang et al, 1971).

Detailed structural and functional studies require an adequate source of protein. Large quantities of TdT are difficult to obtain since kilograms of fresh calf thymus gland are needed, and extensive proteolysis occurs during purification (Deibel et al, 1980 ; Chang et al, 1982). As a result, most of the enzymatic studies reported to date have been done with heterogenous preparations of bovine TdT that contained proteolytic fragments of the enzyme. Cultured cell lines established from patients with acute lymphoblastic leukemia are another rich source of TdT (Deibel et al, 1980). However, the cost of cell production precludes the use of this material for routine mass production of enzyme. Expression of human TdT was also reported, in 1988, in the Baculovirus (Chang et al, 1988) but this system does not appear to be as promising as initially thought and has not been sufficient to permit, ten years later, the full description of the enzyme. Numerous attempts to overexpress TdT in bacteria have failed (Yang et al, 1994 ; Peterson et al, 1985). No crystallographic data are yet available for TdT.

Two murine TdT isoforms were identified, differing in both their ability to add N-regions and their subcellular localization (Bentolila et al, 1995). TdT production being restricted to immature lymphocytes, and the mouse thymus being a small organ, there was no convenient "natural" source for murine TdTs. The authors of the present invention were thus led to express the two murine TdT isoforms in *Escherichia coli* in order to characterize their catalytic properties. The below example describes the optimization of production in modified *E.coli* strains of the short isoform of murine TdT with minimal proteolysis and high specific activity.

### 2. Materials and methods :

*2.1. Expression vectors and bacterial strains--* The cloning into the eukaryotic vector pcDNAI (Invitrogen, San Diego, CA) of the cDNA that encodes the short murine TdT isoform has been described elsewhere (Bentolila, 1995). The full length cDNA was amplified from this pcDNAI derivative by polymerase chain reaction with the Vent-polymerase, using reagent kit from U. S. Biochemical Corp. (Cleveland, OH) and 1 set of primers (Genset) :
1₁: 5'-CCGCGGCATATGGATCCACTGCAAGCAGTCCAC/
1₂: 5'-CCGCGGGAATTCTTAGGCATTTCTTTCCCATGG.

The PCR amplified products were digested with appropriate restriction enzymes, purified on a GTG seaplaque agarose gel (FMC Products) and subcloned into the expression vectors pET22b (Novagen Inc.) and pET28b (Novagen Inc.) at the NdeI/EcoRI sites, using the *E.coli* GT869 strain (Parsot et al, 1986). For expression, pET22b-TdTS and pET28b-TdTS constructs were transformed into the *E.coli* BL21(DE3) cell line (Novagen Inc.) or into a pDC952 containing BL21(DE3) strain. pDC952 is a pACY184 derivative containing the *argU* gene that codes for tRNA^{arg}_{UCU}.

*2.2. Production of murine TdT in E.coli and preparation of the extracts.* Cells transformed with the expression vectors were grown in LB broth from an overnight culture at a 1/400 dilution in the presence of appropriate antibiotics, with vigorous agitation at 37°C, until OD reached 0.8 (about 5 hours). The expression of TdT was induced by the addition of 1 mM IPTG, and the culture was incubated for 3 or 16 hours depending on the temperature (37°C or 15°C, respectively). Cultures at 15°C were grown in a Unitron incubator shaker (InforsHT). Cells were harvested by centrifugation at 5000 rpm for 15 min, resuspended in 1/20 V buffer A (20 mM Tris-HCl pH 8.3, 0.5 M NaCl)-5 mM imidazole and lysed in the French press at 14,000 psi. The lysate was centrifuged in a SS34 rotor for one hour at 10,000 rpm. The pellet was resuspended in buffer A. Aliquots of both pellet and supernatant were supplemented with 1/3 V of 3X SDS sample buffer (150 mM Tris-HCl pH 6.8, 0.03% bromophenol blue, 3% β-mercaptoethanol, 24% glycerol, 6% SDS) and loaded on a 8% SDS acrylamide gel after heating to 70 °C for 5 min. After electrophoretic separation, half of the gel was stained directly with Coomassie Blue. Proteins present in the other half of the gel were transferred onto a nitrocellulose sheet and tested for reaction with a rabbit serum raised against bovine TdT. Immunocomplex was detected by reaction with goat anti-rabbit alkaline phosphatase conjugate followed by development in 100 mM Tris pH 9.5, 100 mM NaCl, 165 µg/ml BCIP, 330 µg/ml NBT. Figure 1, Panel A, lane 1, shows proteins present in *E.coli* BL21 DE3 cells transformed with the pET22b expression vector; lane 2, proteins present in the same cells transformed with the murine TdT c-DNA containing pET22b vector (pET22-TdTS). Panel B, lanes 1 and 2, corresponding immunoreactive material; Coomassie staining (panel C) and immunobloting (panel D) of pellet and supernatant fractions obtained from bacteria transformed with pET22b (lanes 1-2) and pET22b-TdTS (lanes 3-4). Cells were lysed and centrifuged at high speed to pellet the insoluble proteins. Panel C, lane 1 and 3, insoluble proteins and lane 2 and 4, soluble proteins. Panel D, lanes 1-4, corresponding immunoreactive material. Arrows indicate the position of migration of recombinant murine TdT. Molecular weight markers are displayed on the left side of the gels. The percentage of TdT present in the soluble and insoluble fractions was determined by scanning the Coomassie stained gel with a MasterScan I Interpretive densitometer (Scanalytics).

*2.3. TdT purification--* Soluble TdT was purified by affinity chromatography. The French press supernatant was loaded on a 5 ml Hitrap affinity column (Pharmacia) charged with Ni⁺⁺ ions and equilibrated with buffer A-5 mM imidazole. The column was washed with 4 V buffer A-60 mM imidazole. Elution of TdT, with 3 V buffer A-1 M imidazole, was monitored by SDS PAGE analysis. Pure fractions (> 95%) containing TdT were pooled and dialysed against buffer B (20 mM Tris-HCl pH 6.8, 200 mM NaCl, 50 mM MgOAc, 100 mM (NH₄)₂SO₄). Proteins were then concentrated using microsep 10K (Pall Filtron) to a final concentration ranging from 5 to 15 mg/ml and stored at -70°C after addition of 50% glycerol. Protein concentration was estimated by absorption at 280 nm using a theoritical extinction coefficient of 54,870 M⁻¹ cm⁻¹ (Gill et al, 1989).

*2.4. Measurement of TdT activity--* (i) Specific activities and kinetic constants for recombinant murine TdT were obtained using a standard assay that measures the addition of dATP to a (dA)₁₀ oligonucleotide initiator. Enzyme assays were carried out at 35°C by incubating the protein in 200 mM potassium cacodylate, 25 mM Tris-HCl, pH 6.6, 250 mg/ml BSA (from Boehringer), in the presence of 4 mM Mg⁺⁺, 4 µM Zn⁺⁺, with various concentrations of [α ³²P]-dATP (100-20,000 cpm/pmol) and (dA)₁₀, as specified in the legends of the figures. Reactions were stopped at different times with 15 mM EDTA. Aliquots were spotted onto Whatman DE81 discs. Unpolymerized nucleotides were washed away by immersing the discs 3 times (for 5 min) in NH₄COOH/Na₄P₂O₇.10H₂O (300 mM/10 mM), then in H₂O (3 times for 5 min) and briefly in ETOH. Dried discs were counted in a scintillation fluid (Econofluor from Packard). Activity was expressed as the percentage of total dATP present in the reaction that has been incorporated over the time course. Whenever needed, the products of the TdT reactions could be visualized: aliquots taken from the assay described above are simply supplemented with a formamide dye mix (10 mM NaOH, 95% formamide, 0.05% bromophenol blue, 0.05 % xylene cyanole) and loaded, after heat denaturation, onto a 16 % acrylamide denaturating gel and subjected to electrophoresis. Chain length distribution was visualized after exposure of the wet gel under a Kodak film (Biomax MR) for the appropriate time at -70°C. (ii) In the initial experiments, conducted with bacterial extracts and TdT inclusion bodies, only relatively low levels of TdT activity could be detected. Due to the distributive mechanism of TdT polymerization, 1 µM of [5'-³²P] labelled (dA)₁₀ initiator (about one order of magnitude below the Km) was required to visualize substantial chain extension. The (dA)₁₀ primer was labelled with T4 polynucleotide kinase and [α³²P]-ATP and used in the assay at a specific radioactivity of 1500 cpm/pmol. Also, in those experiments, dGTP was used instead of dATP, for polymerized dGTP is known to be more resistant to nucleases likely to be present in the extracts (Deibel et al, 1979).

### 3. Results

*3.1. Production of murine TdT in E. coli.* Since numerous attempts by others to overexpress TdT in bacteria had failed (Yang et al, 1994 ; Chang et al, 1988 ; Peterson et al, 1985), the authors of the present invention decided to explore an array of systems to produce murine TdT. Several constructs were made and tested in appropriate bacterial genetic backgrounds. This section presents a general description of the expression system that was ultimately retained.

The pET expression system (Novagen) uses high copy number vectors that are derivatives of pBR322 with a ColE1 replication origin (Studier et al, 1990). The expression of the recombinant protein is under the control of a T7 promotor and is inducible by IPTG. Depending on the type of vector chosen, the protein can be produced with or without a C-terminal or N-terminal fusion. In addition, the pET system allows straightforward mutational analysis, a property that is needed to address structure-function relationships. The very first attempts to overproduce TdT without fusion, by using the pET22b vector, were successful. Upon induction, TdT was expressed essentially as a polypeptide chain of 58 Kd. This protein was absent in the control experiment that used the pET22b with no TdT c-DNA insert (Fig. 1, Panel A and B). TdT was recovered almost entirely in inclusion bodies when cultures were grown at 37 °C (Fig. 1, Panel C and D). Both soluble and insoluble fractions showed TdT activity whereas no activity was detectable in the control experiment (Fig. 2, panel A).

When looking at the codon content of the murine TdT cDNA, the authors of the present invention noticed a severe skew of codon usage: 32 of the 36 arginine codons in the murine TdT cDNA are rare in *E. coli* , and 22 (AGA/AGG) are the least used of all (frequency of 1.4/2.1 per 1000 codons (Wada et al, 1992). Moreover, they made the observation that rare arginine codons were found three times in tandem in the N-terminal first third of the protein. They also found, in a related experiment, that the bacterial production of mutant proteins in which this portion was deleted was much higher than that of the full length protein. These observations led them to test the hypothesis that the expression in *E. coli* of the *TdT* gene containing many disfavored arginine codons could be improved by overproducing a rare t-RNA (*argU* gene) that reads AGA and AGG (Spanjaard et al, 1990 ; Saxena et al, 1992). Indeed, in these conditions, as shown in Fig. 3, the authors of the present invention observed a 3 fold increase in the production of TdT. In all subsequent experiments, unless otherwise indicated, a derivative of the original bacterial strain was used, that had been transformed with the *argU* gene containing plasmid (pDC952, a derivative of pACY184 compatible with the expression vectors). Error-containing products can sequester and eventually saturate the chaperones that ensure the correct folding of proteins (Kurland et al, 1996).

*3.2. Growing cultures at low temperature allows the production of soluble, active recombinant murine TdT--* Under standard conditions (37°C) the pET expression system did not allow the production of soluble TdT. The authors of the present invention thus investigated the effect of temperature on the production of soluble TdT. Although most of the protein still precipitated in the form of inclusion bodies, a portion was recovered in the soluble fraction when cultures were grown at 25°C. The yield of soluble TdT was significantly improved, up to 80%, when the temperature was further lowered to 15°C, as shown in Fig. 4. This protein was active (Fig. 2, Panel B).

*3.3. Purification of recombinant murine TdT--* In order to facilitate the purification of recombinant murine TdT, the mTdT c-DNA was subcloned into another pET vector (pET28b) that allows the production of a 60 kD fusion protein with an hexa-histidine tag at its N-terminus. The fusion protein could be purified by affinity chromatography in a single step, yielding more than 95 % pure TdT. A typical purification profile is shown in Fig. 5. 5 mg of pure TdT per liter of culture were routinely obtained. The specific activity of the purified protein was around 100,000 units per mg, one unit being defined as one nanomole of dATP polymerized per hour per milligram of protein. The enzyme stored in 50% glycerol at -70 or -20°C for a period of over 6 months showed no detectable decrease in its specific activity.

### Example 2 : Production of soluble, full length murine RAG1 and RAG2 proteins in E. Coli

Proteins RAG1 and RAG2 (coded by *recombination activating 1* and 2 genes) play an essential role in V(D)J recombination. These two proteins had never been expressed in other than their truncated form (Fraser McBlane et al., 1995). When looking at the content of arginine codons in RAG1 cDNA which are rare in *E. coli*, the authors of the present invention found this to be 58/73. Additionally, in the N-terminal portion of RAG1, deleted in the usual constructs, there is a motif of 11 amino acids of which six are arginine, 4 of these corresponding to rare codons. For RAG2 cDNA, 12/18 arginine codons are rare in *E. coli*, and the cDNA corresponding to the usually deleted portion of this protein possesses a tandem AGA-AGA.

The murine RAG1 (M6-3) and murine RAG2 (MR2-1) cDNAs were obtained from M. Oettinger (Schatz et al., 1989 ; Oettinger et al., 1990) and were cloned into the NotI site of pBluescript SK(+) (Stratagene). The full length cDNAs were amplified from these pBS derivatives by polymerase chain rection with the Vent-polymerase, using reagent kit from U. S. Biochemical Corp. (Cleveland,OH) and 2 sets of primers:
1₁: 5'-CCGCGGCATATGGCTGCCTCCTTGCCGTCTACC
1₂: 5'-CCGCGGGTCGACTTAAAACTCCATTGAATCTTGGCT for RAG1, and :
2₁: 5'-CCGCGGCATATGTCCCTGCAGATGGTAACAGTG
2₂: 5'-CCGCGGGAATTCTTAATCAAACAGTCTTCTAAGGAA for RAG2.

The PCR amplified products were digested with appropriate restriction enzymes, purified on a GTG seaplaque agarose gel (FMC Products) and subcloned into the expression vector pET28b (Novagen Inc.) at the NdeI/SalI sites for RAG1 cDNA and at the Nde1/EcoRI sites for RAG2 cDNA, using the *E. coli* GT869 strain (Parsot, 1986) . For expression, pET28b(+)-flRAG1 and pET28b(+)-flRAG2 plasmids were transformed into a pDC952 containing BL21(DE3) strain as above described. pDC952 is a pACY184 derivative containing the *argU* gene that codes for tRNA^{arg} _{UCU}. The production of soluble murine RAG1 and RAG2 in *E. coli* was obtained using conditions already described for murine terminal deoxynucleotidyl transferase.

The authors of the present invention have obtained the expression of the full form of these two proteins in *E. coli.* The authors of the present invention (Figure 6) show that about 80% of RAG1 and 60% of RAG2 are soluble. Lowering the temperature further to 10°C improved the solubility of both proteins.

A review of the cDNA sequences of three human proteins, the catalytic subunit of telomerase (hEST2), CFTR (cystic fibrosis transmembrane conductance regulator protein) and albumin, which, to the inventors' knowledge have not been produced efficiently in their integral form with standard heterologous expression systems, shows them to be rich in the three arginine codons that are the rarest of all codons in *E. coli* (AGG/AGA/CGA): 66 of 125 arginine codons for hEST2, 61 of 78 for CFTR and 21 of 27 for albumin. These are only three of numerous proteins for which minor codons may be adversely affecting protein expression in bacteria and for which the method of the present invention may allow high yields of purified, integral protein.

### REFERENCES

Bagnoli, F., and Lio, P. (1995). Selection, mutations and codon usage in a bacterial model. Journal of theoritical biology *173*, 271-281.
Bentolila, L. A., Fanton d'Andon, M., Nguyen, Q. T., Martinez, O., Rougeon, F. and Doyen, N. (1995) The two isoforms of mouse terminal deoxynucleotidyl transferase differ in both the ability to add N regions and subcellular localization. *EMBO J*. 14, 4221-4229.
Bollum, F. J. (1960) Calf Thymus Polymerase. *J. Biol. Chem.* 235, 2399-2403.
Bollum, F. J. (1974) Terminal deoxynucleotidyl transferase in *The Enzymes,* (Boyer, P. D., ed.), Academic Press, New York, pp. 145-171.
Calderone, T. L., Stevens, R. D., and Oas, T. G. (1996). High-level Misincorporation of Lysine for Arginine at AGA Codons in a Fusion Protein Expressed in *Escherichia coli.* Journal of Molecular Biology 262, 407-412.
Chang, L. M. S. and Bollum, F. J. (1971) Deoxynucleotide-polymerizing Enzymes of Calf Thymus Gland. IV. Homogeneous Terminal Deoxynucleotidyl Transferase. *J. Biol. Chem.* 246, 909-916.
Chang, L. M. S., Rafter, E., Rusquet-Valerius, R., Peterson, R. C., White, S. T. and Bollum, F. J. (1988) Expression and Processing of Recombinant Human Terminal Transferase in the Baculovirus System. *J. Biol. Chem.* 263, 12509-12513.
Coleman, M. S. (1977) Terminal Deoxynucleotidyl Transferase: Characterization of Extraction and Assay Conditions from Human and Calf Tissue. *Arch. Biochem. Biophys.* 182, 525-532.
Deibel, M. R. and Coleman, M. S. (1979) Purification of a high Molecular Weight Human Terminal Deoxynucleotidyl Transferase. *J. Biol. Chem.* 254, 8634-8640.
Deibel, M. R. and Coleman, M. S. (1980) Biochemical Properties of Purified Human Terminal Deoxynucleotidyltransferase. *J. Biol. Chem.* 255, 4206-4212.
Deibel, M. R. and Coleman, M. S. (1980) Limited Proteolysis of Calf Thymus Terminal Deoxynucleotidyl Transferase. *Arch. Biochem. Biophys.* 202, 414-419.
Doyen et al, (1993) Differential splicing in mouse thymus generates two forms of terminal deoxynucleotidyl transferase, *Nucleic Acids Research,* 1993, vol. 21, n° 5, 1187-1191
Garcia, G.M., P.K. Mar, D.A. Mullin, J.R. Walker, and N.E. Prather (1986) The *Escherichia coli dna*Y gene encodes an arginine transfer RNA. Cell 45:453-459.
Gill, A. B. C. and von Hippel, P. H. (1989) Calculation of protein extinction coefficients from amino acid sequence data. *Anal. Biochem.* 182, 319-326.
Kane, J. F. (1995) Effects of rare codon cluster on high-level expression of heterologous proteins in *Escherichia coli. Curr. Opin. Biotechnol.* 494-500.
Kirch et al, (1998) Dual role of RAG2 in V(D)J recombination : catalysis and regulation of ordered Ig gene assembly. *The EMBO Journal.* vol. 17, n° 16, 4881-4886.
Kurland, C. and Gallant, J. (1996) Errors of heterologous protein expression. *Curr. Opin. Biotechnol.* 7, 489-493.
La Vallie, E. R., DiBlasio, E. A., Kovacic, S., Grant K. L., Schendel, P. F. and McCoy, J. M. (1993) A Thioredoxin Gene Fusion Expression System That Circumvents Inclusion Body Formation in *the E.coli* Cytoplasm. *Bio/tech.* 11, 187-193.
Fraser Mc Blane, J., van Gent, D. C., Ramsden, D. A., Romeo, C., Cuomo, C. A., Gellert, M., and Oettinger, M. A. (1995). Cleavage at a V(D)J recombination signal requires only RAG1 and RAG2 proteins and occurs in two steps. Cell *83*, 387-395.
Oettinger, M. A., Schatz, D. G., Gorka, C., and Baltimore, D. (1990). RAG-1 and RAG-2, adjacent genes that synergistically activate V(D)J recombination. Science *248*, 1517-1523.
Olins, (1996), Expression systems quantity versus authenticity of heterologous produced proteins : an inevitable compromise ? *Current Opinion in Biotechnology*, 7:487-488
Parker, J. (1989). Errors and alternatives in reading the universal genetic code. Microbiological Review *53*, 273-298.
Parsot, C. (1986). Evolution of biosynthetic pathways: a common ancestor for threonine synthase, threonine dehydratase and D-serine dehydratase. The EMBO Journal *5*, 3013-3019.
Peterson, R. C., Cheung, L. C., Mattaliano, R. J., White, S. T., Chang, L. M. S. and Bollum, F. J. (1985) Expression of Human Terminal Deoxynucleotidyl Transferase in *Escherichia coli. J. Biol. Chem.* 260, 10495-10502.
Pouwels et al (1985 s upp. 1987) cloning vectors : a Laboratory Manual
Roman et al, (1997), Complementation of V(D)J recommandation Deficiency in RAG^{-1/-} B cells reveals a requirement for novel elements in the N-terminus of RAG-1, *Immunity,* vol. 7, 13-24
Ramsden et al, (1997), Cell-free V(D)Jrecombination, *Nature*, vol. 388, 31
Saxena, P. and Walker, J. R. (1992) Expression of *argU*, the *Escherchia coli* gene coding for a rare Arginine tRNA. *J. Bacteriol*. 174, 1956-1964.
Schatz, D. G., Oettinger, M. A., and Baltimore, D. (1989). The V(D)J recombination activating gene, RAG-1. Cell *59*, 1035.
Schein, C. H. (1989) Production of soluble recombinant proteins in bacteria. *Bio/tech.* 7, 1141-1149.
Spanjaard R.A. and van Duin, J. , (1988), Proc. Natl. Acad. Sci. USA *85*, 7967-7971
Spanjaard, R. A., Chen, K., Walker, J. R. and Duin, J. v. (1990) Frameshift suppression at tandem AGA and AGG codons by cloned tRNA genes: assigning a codon to *argU* tRNA and T4 tRNAarg. *Nucleic Acids Res.* 18, 5031-5036.
Wada, K., Wada, Y., Ishibashi, F. and Ikemura, T. (1992) Codon usage tabulated from the genebank genetic sequence data. *Nucleic Acids Res.* 20, 2111-2118.
Yang, B., Gathy, K. N. and Coleman, M. S. (1994) Mutational Analysis of Residues in the Nucleotide Binding Domain of Human Terminal Deoxynucleotidyl Transferase. *J. Biol. Chem.* 269, 11859-11868.
Zhang, S., Zubay, G., and Goldman, E. (1991). Low-usage codons in *Escherichia coli*, yeast fruit fly and primates. Gene *105*, 61-72.

## Claims

1. A method for producing a soluble integral heterologous protein in a bacterium,
wherein said protein contains amino acids encoded by codons that are rare in the genome of said bacterium and said bacterium does not naturally contain a sufficient amount of tRNA corresponding to said rare codons to allow the production of said complete heterologous protein ;
said method comprising the steps of :
a) transforming said bacterium with i) a nucleic acid encoding at least a tRNA that is rare in said bacterium and that corresponds to at least said rare codon, so as to overexpress said tRNA ; and ii) a nucleic acid encoding said heterologous protein ;
b) culturing said bacterium at a temperature inferior to the usual growth temperature, whereby said heterologous protein is produced in an integral and soluble form ;
c) extracting said heterologous protein from said bacterium ; and
d) optionally purifying said heterologous protein.

2. The method of claim 1, wherein the step of culturing the bacteria is carried out at a temperature of not more than 20°C.

3. The method of claim 2, wherein the step of culturing the bacteria is carried out at a temperature of not more than 15°C.

4. The method of any of claims 1 to 3, wherein the step of culturing the bacteria is carried out at a temperature of not less than 4°C.

5. A method according to any of claims 1 to 4, wherein the step of culturing the bacteria is carried out at a temperature between 4°C and 20°C.

6. A method according to any of claims 1 to 5, wherein said bacterium is *Escherichia coli.*.

7. A method according to any of the preceding claims, wherein said heterologous protein is terminal deoxynucleotidyl transferase (TdT), or variants thereof.

8. A method according to claim 7, wherein said transformed bacterium is the *E. coli* strain identified under the deposit number CNCM I-1981 or CNCM I-1982.

9. A method according to any of claims 1 to 6, wherein said heterologous protein is RAG 1 or RAG2, or variants thereof.

10. A method according to claim 9, wherein said transformed bacterium is the *E. coli* strain identified under the deposit number CNCM I-2086 or CNCM I-2087, respectively producing murine RAG1 and murine RAG2.

11. A recombinant soluble and integral TdT protein produced in bacteria.

12. A recombinant soluble and integral RAG1 or RAG2 protein produced in bacteria.
